# EUROPEAN PATENT SPECIFICATION

(11) **EP 4 103 892 B1**
(45) Date of publication and mention of the grant of the patent: **06.08.2025**
(21) Application number: 21703493.3
(22) Date of filing: 11.02.2021
(51) Int. Cl.: F24F 8/10, F24F 8/30

(54) **AIR PURIFIER**
LUFTREINIGER
PURIFICATEUR D'AIR

(30) Priority: 14.02.2020 IN 202021006451; 14.02.2020 IN 202021006450; 14.02.2020 IN 202021006452; 14.02.2020 IN 202021006453; 14.02.2020 IN 202021006449; 30.03.2020 EP 20166870; 26.05.2020 EP 20176484
(43) Date of publication of application: 21.12.2022
(73) Proprietor: Blueair AB, 115 26 Stockholm (SE)
(72) Inventor: DUNBERGER, Lars Henrik, 115 26 Stockholm (SE); NETHAJI, Alagirisamy, Mumbai 400099 Andheri (IN); WENNERSTRÖM, Johan Daniel, 115 26 Stockholm (SE)
(74) Representative: Newbould, Frazer Anthony
(86) International application number: PCT/EP2021/053332
(87) International publication number: WO 2021/160735

(56) References cited:
- EP-A2- 1 245 289
- WO-A1-2011/030603
- WO-A1-2018/058716
- WO-A1-2020/007549
- CN-A- 105 823 131
- JP-A- 2012 223 324
- US-A1- 2007 034 082

## Description

The present invention relates to an improved air purifier.

US 2007 034 082 discloses an air purifier including an ionizing assembly that operates to charge particulate material in an air flow passing through the purifier. The charged particulate material is attracted to and retained by a filter element disposed downstream of the ionization assembly and having an electrical charge opposite to the charged particulate material. The purified air passing through the filter is directed out of the device, optionally in conjunction with a fragrance that is added to the purified air flow. The ionizing assembly is formed with a ground member disposed adjacent the ionizing member to keep the electrons generated by the ionizing assembly within the purifier, and thus prevent static discharges from occurring outside of the purifier. The air flow is directed by a fan through the purifier in an angular and substantially laminar manner, such that the efficiency of the purifier is increased.

US 2002 141 131 discloses an improved air ionizer apparatus includes an air inlet, a high voltage source, an electrode electrically connected to the high voltage source for generating ions and an air outlet. An air mover is provided for causing air to flow into the air ionizer through the air inlet and out of the air ionizer through the air outlet. A foraminous filter comprising an electrically conductive material is electrically coupled to at least one of a voltage source and ground. The filter is positioned over at least one of the air inlet, the air outlet and the electrode, such that air flowing into the air inlet, air flowing out of the air outlet or air flowing past the electrode flows through the filter. In a preferred embodiment, the filter comprises a metal grid or screen.

CN 105 823 131 discloses a fresh air purification combined system for a teaching area. The fresh air purification combined system comprises an air treatment assembly. The air treatment assembly comprises a fresh air ventilator and an air purifier. The fresh air ventilator and the air purifier are arranged independently and fixed to different positions in a room, and thus the indoor air quality can be controlled through combination. According to the fresh air purification combined system for the teaching area, through the combination of the constant-oxygen air purifier and the purification type fresh air ventilator, a constant-clean, constant-oxygen, energy-saving and green ecological classroom can be created, and a safe and clean study environment is provided for students.

WO 2018/058716 discloses an all-in-one fresh air purifier, comprising a housing (1), an indoor return air inlet (21), an outdoor fresh air outlet (22), a fresh air delivery outlet (2), an indoor return air discharge outlet (12) and a power supply control device provided in the housing (1); provided in sequence in the housing (1) are a stage one filter (3), a heat exchange core (4), an exhaust fan (5), a stage two filter (6), an ion cloud dust removal module, an air blower (9) and a stage three filter (10); the exhaust fan (5), ion cloud dust removal module and air blower (9) are electrically connected to the power supply control device. The present all-in-one device combines ventilation and air purification functionality, is plug-and-play, presents no difficulties in installation and maintenance or structural problems of installing air pipes and damaging a room, has high negative ion generation, has a long conveying distance, has strong dust removal and disinfection effects, and furthermore does not generate ozone during operation, maintaining a healthy environment.

WO 2020/007549 discloses an ioniser for an air purifying device wherein the ioniser comprises a corona discharge tip and is capable of alternately generating positive corona discharge and a negative corona discharge, wherein the ioniser comprises a voltage source, a switch for switching from a first polarity to a second polarity or vice versa during use and a timer for timing the time interval between switching from a first polarity to a second polarity and wherein the switch is activated to switch the polarity after a period of from 0.2 to 20 seconds. Air purifying device comprising such an ioniser, a fan and a filter and wherein the ioniser is disposed after the fan and before the filter in an air flow direction. The invention also relates to a vehicle comprising such an ioniser and a dwelling comprising such an air purifying device.

JP 2012 223324 and WO 2011/030603 disclose air purifying devices upon which the preamble of appending claim 1 is based.

Despite the prior art there remains a need for improved air purifiers, in particular air purifiers which are more hygienic throughout their working life.

Air purifiers work by filtering ambient air through a filter. Accordingly, anything in the air is in theory capturable by the filter. While there are different types of filtration means, from particulate filter to gas filter, it is a necessary consequence of the functioning of an air purifier that they also capture microbes caught in the air flow.

It is also a routine feature of air purifiers that the main focus is removing pollution from the ambient air and various sensors which indicate that particulates are being removed exist. Consequentially, it also is quite routine for an air purifier to work in an automatic mode whereby the presence of particulates influences the air flow speed through the device. Thus, when the air quality is good, it is possible and often desirable that the purifier is maintained in idle or stand-by mode to conserve energy.

However, when air is not passing through the purifier micro-organisms caught by the filter are able to grow quickly and form biofilms which can have a deleterious effect on effective filter life span and also cause a health hazard to the users who habit the domestic setting.

According to the invention there is provided a method for sterilising a filter media in an air purifier by:
(A) subjecting said filter media to an air flow;
(B) subjecting said filter media to an ion cloud; and
where steps (A) and (B) are in any order and whereby (A) and (B) are carried out exclusively.

We have surprisingly found that the air draft required to kill micro-organisms is significantly lower than that required for air filtration. Further, we have found that subjecting a surface to an air draft and then an ioniser, or *vice* versa, provides enhanced sterilisation characteristics.

Preferably, the air purifier comprises removable filter media which may be particulate filter or gas filter.

The air flow speed measured at the removable filter is known in the art as the media velocity. Media velocity is the velocity at which the air travels through the filter. Media velocity has to be controlled perfectly to ensure that the maximum amount of particles are trapped. Too fast and many of the pollutants fly straight through unfiltered. Too slow and the purifier is not reaching the farthest corners of your room quickly enough to be effective.

Preferably, the air flow (A) speed (media velocity) measured at the removable filter is at least 0.1 cms⁻¹ measured at the filter medium. The measurement at the filter medium is taken from the spatial centre point on the fan side of the filter media surface. Where there is more than one filter medium, the one taken for the air draft measurement is the one which is closest to the air flow generator and so receives the air draft first.

More preferably, the air flow speed measured at the removable filter is from 0.1 to 2.5 cms⁻¹. Most preferably, the air flow speed measured at the removable filter is from 0.8 to 1.2 cms⁻¹.

The ioniser used comprises an emitter electrode and a ground electrode. The emitter electrode discharges an ion cloud between the emitter and the receiver on application of a suitable voltage, preferably from -10 to 10kV but more preferably around the range -8 to 8kV.

The emitter electrode is preferably a point, tip or multiple tips or points for example a brush and is in electrical communication with a voltage source. Preferably, the emitter electrode is within 20cm, more preferably 15cm, especially preferably 10 cm and most preferably up to 5 cm from the nearest filter medium to be sterilised by bathing it in an ion cloud during activation of the ioniser when required. This distance is the distance from the tip of the emitter electrode to the nearest part of nearest filtration medium, more specifically the part of the filter medium which filters rather than for example a structural frame.

The receiving electrode is preferably a metallic part in the shape of a ring so that the resulting ion cloud is in three dimensions as the ions are spent away from the emitter and are then pulled towards the receiver. Preferably, the receiving electrode is in the form of a cage which extends away from the emitting electrode in an air flow direction. More preferably, the receiving electrode is in the form of a reticulated arrangement and which extends towards the filter. Such extension may present a hemispherical or partial cylindrical shape such that the receiving electrode is downstream in an air flow direction from the emitting electrode.

Preferably, the emitter is centrally disposed between a receiver in the form of a ring. The emitter may be disposed pointing towards or away from the air flow during use but it is preferred that the emitter is pointed towards the surface to be sterilised, for example, an internal wall or a filter medium.

Preferably, the air flow generator generates an air flow commensurate with sterilisation of an internal surface of the air purifier and/or a filter media for a period of from 1 second to 10 hours.

According to the invention, (A) and (B) are carried out exclusively. By this is meant that the surface to be sterilised is first subjected to an air draft and then when the air draft is stopped, the surface is exposed to a burst of ionisation. Similarly, the ionisation may come before the air draft.

It is also preferred that the sequence of air draft and then ionisation, or vice versa, is repeated such that the surface to be sterilised is subjected to both air draft and ionisation on an intermittent basis.

Preferably, (A) has a duration of from 1 min to 10 hours

Preferably, (B) has a duration of from 1 min to 10 hours.

In a further aspect there is provided an air purifier comprising a removable particulate or gas filter, an air flow generator, a means for controlling said air flow generator, a first air flow setting with an air filtration air flow speed and a second air flow setting which correlates with sterilisation of an internal surface of the air purifier and/or removable particulate or gas filter and an ioniser.

Preferably, the air purifier comprises an operable mode for sterilising an internal surface or a filter medium and whereby the air draft corresponds with said second air flow setting and this is activated either before or after a period of ionisation.

We have surprisingly found that a combination of a modest air flow rate with ionisation treatment can prevent growth of micro-organisms and drastically reduce the prevalence of live micro-organisms being blown into the ambient air and also released into the environment during filter change.

Such micro-organisms include gram positive bacteria, gram negative bacteria, spores, moulds and fungi as well as any viruses within said micro-organisms.

We have surprisingly found that by providing a low speed air draft and then a burst of ionisation, or *vice versa,* we can drastically reduce micro-organism viability on the internal surfaces of the air purifier and in particular on the filter media. While the purifier is operating there is no increased risk of micro-organisms growing and multiplying on the filter. However, when it is in idle or stand-by mode and the conditions are favourable, the micro-organisms grow.

In preferred embodiments of the invention the purifier ascertains the likelihood of conditions conducive to micro-organism growth and when such conditions are deemed to exist it actuates the air flow generator and/or the ioniser to destroy the microbes on the filter, or even those on the internal surfaces of the purifier.

When the processor determines that the conditions are conducive to micro-organism growth, for example with reference to inputs from a temperature sensor and a humidity sensor and then with reference to an appropriate look-up table, it either provides an indication, for example by way of a visual or audible signal, or electronically to a remote device such as a mobile phone so that the user is notified that the air flow generator should be employed, or it automatically actuates the fan or impeller at a low speed as described herein, or the ioniser, and which is sufficient to prevent micro-organism growth or to directly destroy the micro-organisms. It is important to note that this activation of the ioniser or fan is appropriate only when the device is in stand-by mode or idle. When the ioniser is functioning or when the fan is functioning no micro-organism can grow on the filter.

Preferably, the purifier has first mode in which the choices are either: no action, where the conditions determined by the humidity sensor and temperature sensor are such that no or low micro-organisms growth is anticipated; an alert by way of an electronic signal to a mobile device to alert the user that conditions are favourable to micro-organisms and permitting the option for the user to actuate the fan; and a warning level where the user is warned that micro-organism growth is likely and strongly recommending to the user to actuate the fan or impeller, or the ioniser.

A second mode may operate similarly in that indications are made determined by the input from the temperature and humidity sensors but instead of a warning or an alert, the machine is automatically turned on when conditions are such that micro-organism growth is likely.

The user of course may select one of these two modes where appropriate.

When the processor determines that the conditions are conducive to micro-organism growth it either provides an indication, for example by way of a visual or audible signal, or electronically to a remote device such as a mobile phone so that the user is notified that the air flow generator and/or ioniser should be employed.

Temperature sensors are known in the art and are commercially available from Sensirion. Suitable examples of temperature sensors include STS3x series.

Humidity sensors are known in the art and are commercially available from Sensirion. Suitable examples of humidity sensors include SHT3x series.

In a preferred embodiment the means for controlling the air flow generator and/or ioniser based on input from said sensors is conducted automatically, for example by a processor. In such an embodiment the sensors sense the temperature and/or humidity on a continuous or intermittent basis and send information back to the processor. The processor determines whether the conditions are conducive to micro-organism growth based on at least temperature or humidity. Preferably, the processor determines whether the conditions are conducive to micro-organism growth based on temperature and humidity. More preferably, the processor determines the likelihood of micro-organism growth additionally based on parameters such as geographical location, time of the day, week, month or season or even the pollution levels as well as any specific conditions that occur, for example virus pandemics or bush fires, and combinations of any of these.

For example, in Asia, the wet seasons are typically defined by the monsoon and occurs in the summer. In contrast the summer in Europe and North America is characterised by drier weather. Similarly, the hemispheres have different seasonal characteristics.

Preferably, the geographical location is determined by GPS or through the purifiers WIFI capability. It may also be provided by way of user input during a set-up process.

The purifier is powered by any suitable power source including internal sources, e.g. batteries, and external power sources. The power is used to drive a motor which in turn powers at least the air flow generator and the ioniser where present.

Preferably, the filter media comprises at least one of carbon, activated carbon, a non-woven, a thermoplastic, a thermosetting material, a porous foam, fibreglass, paper, a high loft spunbound web, a low loft spunbound web, a meltblown web and or a bi-modal fiber diameter meltblown media.

Preferably, the removable particulate filter is a High Efficiency Particulate Air (HEPA) filter. It is to be understood that while the filter part of an air purifier is a vital part of its function, air purifiers are not commonly manufactured with a filter in place. They are practically always manufactured separately and most importantly often by a different commercial enterprise than of the manufacturer of the air purifier itself. It is also typical for a manufacturer of filters to manufacture filters for different air purifier models made by different manufacturers. The particulate filter is to be contrasted with the pre-filter or any dust filter which is present. Pre-filters and dust filters are not considered HEPA filters as they do not have the particulate capturing capability exhibited by HEPA filters. Preferably, the filter is precharged before application to the air purifier.

Pre-filters are filters which have a low air resistance and also function as a poke guard, preventing the user from touching the volute or impeller assembly. The pre-filters are not intended to exhibit any major effect in the context of air purification. They do not have the air resistance or particle entrainment capability of dedicated particulate filters. Preferably the pre-filter is not a HEPA filter.

The purifier of the inventions also comprises a fan or impeller. The fan may be a bladeless fan, an axial fan but it is preferred that the fan is a radial fan.

Preferably, the air purifier comprises an ioniser. Preferably, the ioniser comprises a corona discharge tip and a receiving electrode. When the corona discharge tip is subjected to an appropriate electric voltage it generates an ion cloud between the tip and the receiving or ground electrode.

The ioniser is preferably disposed on the interior of said purifier. However, it is also preferred that the purifier comprises an ioniser additionally disposed on the exterior of said device. More preferably, it is preferred that any external ioniser is disposed at the top of the device. Locating the external ioniser at the top of the device means that domestic dust particles are ionised as they fall through the air towards the ground and are therefore more likely to aggregate as they become charged. As they become more aggregated they are more easily caught up in the air circulation pattern created by the device and so more easily filtered.

Where the ioniser is disposed in the interior of the device it is preferred that it is located before the removable particulate filter in an air flow direction.

Preferably, the device comprises an interior ioniser and an exterior ioniser. The exterior ioniser facilitating aggregation of domestic dust particles and the interior ioniser facilitating capture of the aggregated dust particles by the removable particulate filter. In both instances the ionisation permits less dense filtration media and low air speed (fan) speeds.

### EXAMPLE 1

The following experiment sets out to assess the impact of ionisation alone on micro-organism viability on a substrate, in this case a particulate filter. The ioniser was subjected to -5kV in order to emit an ion stream.

The air flow applied to the substrate in this experiment was zero.

The micro-organisms used were *Staphylococcus aureus* and *Pseudomonas aeruginosa* and the incubation period to generate the biofilm was 5 days.
Results:

| **Exposure time (h)** | **1h** | **2h** | **4h** |
|---|---|---|---|
| Log reduction microbes | 0.48 | 0.78 | 1.16 |

| **Distance (cm)** | **5** | **10** | **15** |
|---|---|---|---|
| Log reduction microbes | 0.75 | 0.5 | 0.28 |

Conclusion:
The closer the ioniser emitter to the substrate the better the impact on micro-organism viability. Further, a burst of ionisation around 2 hours performs most optimally.

### EXAMPLE 2

The following experiment sets out to assess the impact of a low air draft alone on micro-organism viability on a substrate, in this case a particulate filter. No ionisation of the substrate took place.

The micro-organisms used were *Staphylococcus aureus* and *Pseudomonas aeruginosa* and the incubation period to generate the biofilm was 5 days.

Results:

| **Air flow rate (ms⁻¹)** | **0.018** | **0.4** | **1** |
|---|---|---|---|
| Log reduction microbes | -0.75 | 0.52 | 1.2 |

A low air draft of 0.018ms⁻¹ resulted in no reduction of micro-organism growth though this still resulted in some slowing down of growth.

### EXAMPLE 3

An air purifier was set up such that a particulate filter was subjected to ionisation as well as a low air draft (1.0 cms⁻¹). The air draft was for 10 hours and the ionisation for 2 hours with the ionisation step coming first.

Again, the ioniser was set at -5kV and the test micro-organism (*Staphylococcus aureus* and *Pseudomonas aeruginosa*) was allowed to grow for 5 days prior to the test.

The control showed log micro-organism count of 6 while the test score was 2.5.

### EXAMPLE 4

An air purifier was set up such that an interior surface of the purifier was subjected to ionisation as well as a low air draft (1.0 cms⁻¹). The air draft was for 10 hours and the ionisation for 2 hours with the ionisation step coming first.

Again, the ioniser was set at -5kV and the test micro-organism (*Staphylococcus aureus* and *Pseudomonas aeruginosa*) was allowed to grow for 5 days prior to the test.

The control showed 152 colonies of micro-organism while the test score showed 6.

## Claims

1. A method for sterilising filter media in an air purifier by: (A) subjecting said filter media to an air flow; (B) subjecting said filter media to an ion cloud; **characterized in that** steps (A) and (B) are in any order and whereby (A) and (B) are carried out exclusively.

2. A method according to claim 1 wherein the air purifier comprises removable filter media and where the air flow speed during step (A) measured at the removable filter media is from 0.1 to 1.2 cms⁻¹.

3. A method according to any preceding claim wherein (A) has a duration of from 1 min to 10 hours.

4. A method according to any of claims 1 to 3 wherein (B) has a duration of from 1 min to 10 hours.

5. A method according to any preceding claim wherein said air purifier comprises a removable particulate or gas filter, an air flow generator, a means for controlling said air flow generator, a first air flow setting with an air filtration air flow speed and a second air flow setting which correlates with sterilisation of an internal surface of the air purifier and/or removable particulate or gas filter.

## Patentansprüche

1. Verfahren zum Sterilisieren von Filtermedien in einer Luftreinigungseinrichtung durch: (A) Bewirken, dass die Filtermedien einer Luftströmung ausgesetzt sind, (B) Bewirken, dass die Filtermedien einer Ionenwolke ausgesetzt sind; **dadurch gekennzeichnet, dass** die Schritte (A) und (B) in einer beliebigen Reihenfolge erfolgen und (A) und (B) exklusiv ausgeführt werden.

2. Verfahren nach Anspruch 1, wobei die Luftreinigungseinrichtung entfernbare Filtermedien umfasst und wobei die Luftströmungsgeschwindigkeit während Schritt (A), die an den entfernbaren Filtermedien gemessen wird, im Bereich von 0,1 bis 1,2 cm/s liegt.

3. Verfahren nach einem vorhergehenden Anspruch, wobei (A) eine Dauer im Bereich von 1 min bis 10 Stunden aufweist.

4. Verfahren nach einem der Ansprüche 1 bis 3, wobei (B) eine Dauer im Bereich von 1 min bis 10 Stunden aufweist.

5. Verfahren nach einem vorhergehenden Anspruch, wobei die Luftreinigungseinrichtung einen entfernbaren Partikel- oder Gasfilter, einen Luftströmungsgenerator, ein Mittel zum Steuern des Luftströmungsgenerators, eine erste Luftströmungseinstellung mit einer Luftfiltrierungs-Luftströmungsgeschwindigkeit und eine zweite Luftströmungseinstellung, die mit der Sterilisation einer Innenfläche der Luftreinigungseinrichtung und/oder des entfernbaren Partikel- oder Gasfilters korreliert, umfasst.

## Revendications

1. Procédé pour stériliser un milieu filtrant dans un purificateur d'air par : (A) soumission dudit milieu filtrant à un courant d'air ; (B) soumission dudit milieu filtrant à un nuage ionique ; **caractérisé en ce que** les étapes (A) et (B) sont dans n'importe quel ordre, et moyennant quoi (A) et (B) sont effectuées exclusivement.

2. Procédé selon la revendication 1, dans lequel le purificateur d'air comprend un milieu filtrant amovible et où la vitesse d'écoulement de l'air pendant l'étape (A), mesurée au niveau du milieu filtrant amovible, est de 0,1 à 1,2 cm.s⁻¹.

3. Procédé selon l'une quelconque des revendications précédentes, dans lequel (A) dure de 1 minute à 10 heures.

4. Procédé selon l'une quelconque des revendications 1 à 3, dans lequel (B) dure de 1 minute à 10 heures.

5. Procédé selon l'une quelconque des revendications précédentes, dans lequel ledit purificateur d'air comprend un filtre à gaz ou à particules amovible, un générateur de courant d'air, un moyen pour commander ledit générateur de courant d'air, un premier réglage d'écoulement de l'air avec une vitesse d'écoulement de l'air de filtration d'air et un deuxième réglage d'écoulement de l'air qui est corrélé à la stérilisation d'une surface interne du purificateur d'air et/ou du filtre à gaz ou à particules amovible.
